Europäisches Patentamt

European Patent Office

Office européen des brevets

(11) Publication number: **0 223 604 B1**

# EUROPEAN PATENT SPECIFICATION

(45) Date of publication of patent specification: **11.03.92**  (51) Int. Cl.⁵: **A61M  3/02**

(21) Application number: **86309042.9**

(22) Date of filing: **19.11.86**

(54) **A water bag for irrigating the colon.**

(30) Priority: **19.11.85 DK 5330/85**

(43) Date of publication of application:
**27.05.87 Bulletin  87/22**

(45) Publication of the grant of the patent:
**11.03.92 Bulletin  92/11**

(84) Designated Contracting States:
**DE ES FR GB IT NL SE**

(56) References cited:
**DE-A- 2 515 889**
**FR-A- 2 426 249**
**FR-A- 2 522 143**
**GB-A- 2 145 224**
**US-A- 4 525 156**

(73) Proprietor: **COLOPLAST A/S**
**4, Bronzevej**
**DK-3060 Espergaerde(DK)**

(72) Inventor: **Ballan, Akeel**
**Pragtstjernevej 1**
**DK-2400 Copenhagen NV(DK)**

(74) Representative: **Piesold, Alexander James et al**
**Frank B. Dehn & Co. Imperial House 15-19 Kingsway**
**London WC2B 6UZ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

# Description

The present invention relates to a reusable water reservoir bag of a flexible plastic sheet material for use in irrigating the colon of colostomy patients.

It is very common for colostomy patients to wear a bag for collection of feces, which are discharged into the bag continuously or at more or less regular intervals. In other cases, however, the ostomy opening is closed by a tampon, a stopper, or some similar closure means, optionally magnetically held by means of suitable magnetic means.

In such cases it is desirable to empty the colon mechanically by the aid of aqueous enemas. Such an emptying is initiated by distending the large intestine mechanically by pouring into it water, most frequently in quantities of l to l.5 litres. This causes a reflex emptying of the colon and experience has shown that by suitable intervals between the enemas - varying from patient to patient and of for instance 24, 48 or even 72 hours - one can obtain a situation in which the intestine only empties itself at predetermined times.

For obvious reasons it is desirable that the emptying of the intestine takes place as quickly as possible when first started. It has been found that the temperature of the water used as the enema has great importance for the speed of the emptying. In order to obtain an optimum emptying rate the temperature of the water poured into the intestine to irrigate it must be about 37°C, i.e. body temperature, and deviations from this of as little as about 2 to 4°C may have a great effect on the efficiency and speed of the emptying of the intestine. If the water is too cold, painful attacks of spasms can occur in and around the intestine and the speed of emptying is decreased. If the water is too hot, a relaxation of the musculature of the intestine takes place and so the speed at which the intestine is emptied also decreases.

The water employed as aqueous enema for the colon of colostomy patients is usually fed from a plastic bag provided with a tube which is put into connection with the ostomy opening by means of, e.g., a catheter or a conus. However, existing irrigation bags are not provided with any means for indicating the temperature of the water and the user has to estimate the temperature of the water by touching the filled bag, e.g. with an elbow or palm or better the back of a hand. Such a temperature estimate is however very unreliable and can easily involve a misjudgement so that even water with a temperature 7-l0°C too low can be estimated as being sufficiently warm whereas a temperature of e.g. 40°C is very likely to be estimated as too high.

From US patent specification No. 4,525,l56 there is known a sanitary disposable gastric lavage closed system including a flexible liquid reservoir bag wherein there is placed a thermometer. A similar closed system is said to be usable for administering enemas and colonic irrigations. The patent specification does not describe the thermometer but its Fig. l clearly shows it to be an ordinary glass thermometer.

Such a thermometer is not very practical for use in a resuable water reservoir of a flexible plastic sheet material, especially so because it is desirable to keep the bag in a flat condition between the uses thereof, which is not really possible with a glass thermometer unless it is mechanically strong, which will add to its volume and cost.

It is an object of the invention to provide a water reservoir bag in which the problems associated with a glass thermometer are generally reduced or eliminated.

According to the invention there is provided a reusable water reservoir bag of a flexible plastic sheet material for use in irrigating the colon of colostomy patients characterized in that it is provided with a thin thermometer comprising encapsulated liquid crystals, the thermometer being intimately and permanently attached to the outside of the bag and being adapted to register the temperature of the water in the bag in the range of 37°C ± 10°C.

In commerce there are a series of thin, flat thermometers which are flexible and may be adhered or even welded to plastic sheet surfaces. Some of the commercial thermometers comprising encapsulated liquid crystals show the temperature in the usual manner with numbers, others indicate the temperature within narrow ranges, e.g. within ranges of each 2°C, by means of colour reactions (colour change). In preferred embodiments of the invention the latter type is preferred because they are easier to read and an accuracy of 2°C is sufficient for the present purpose.

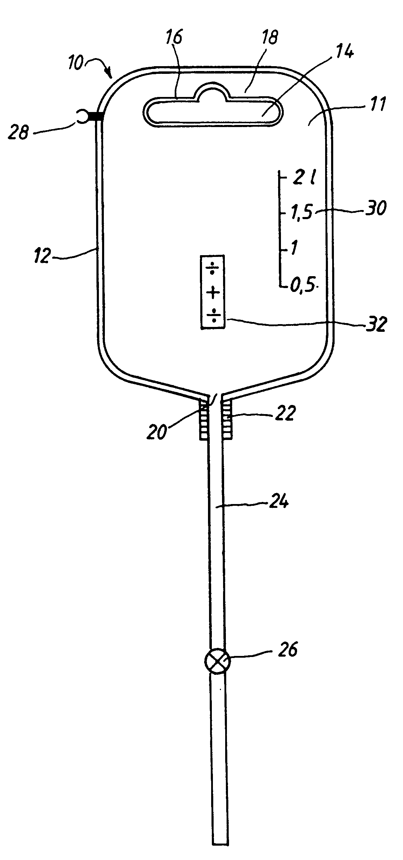

A preferred embodiment of the invention will now be described by way of example and with reference to the drawing which shows a water reservoir bag seen from one side.

A water bag 10 according to the invention can, apart from the thermometer, be of any known kind. In the embodiment shown it has two walls 11 welded together in a marginal seam 12. In the upper part both of the walls have an opening 14 and the portions of the bag adjacent this opening also have a welding seam 16.

The opening makes the upper portions 18 of the bag usable as a handle and for suspension of the bag when it is to be put into use. The suspension may for instance be at shoulder height of the user.

In its lower part the bag has an opening 20 with a diameter in the distended state of the bag of, e.g., about 5 mm. The opening 20 opens into a spout 22 to which there is secured, e.g. by hot melting, a tube 24 provided with a valve 26. The tube 24, having a diameter of e.g. 6-7mm, serves for passing water from the reservoir to an ostomy opening. The valve 26 may be closed or partially or fully open whereby it is possible to regulate the rate at which water flows from the bag l0 to the ostomy opening. The tube 24 and the opening 20 may optionally also be used for filling the reservoir with water having a suitable temperature, but there may also be a separate closable inlet opening in the top of the bag.

The bag l0 may be provided with a holding means 28 for holding the tube when the bag is not in use. It may also be provided with a volume graduation 30 so that the patient may easily see when a suitable amount of water has flowed into the large intestine.

The particular property of the bag according to the preferred embodiment of invention is a thin usually flexible thermometer 32 of the kind with encapsulated liquid crystals. The thermometer has been adhesively affixed with its back surface to one of the walls ll of the bag. In the embodiment shown the thermometer has no numbers but on the contrary is adapted to indicate a range of the temperature of the water in the bag by colour change, e.g. a range of 2°C. When the temperature of the water is in the correct range (i.e. about 36° to 38°C), the colour corresponding to this range is visible and shows the symbol +. The symbols above and below (in the position of the thermometer shown; it might equally well be horizontally placed) the symbol + are provided to show that the temperature is outside the desired range, these symbols being shown by colours corresponding to their respective upper and lower ranges.

However, many other readily registrable forms of indicating whether the temperature is correct or wrong are conceivable.

When the reservoir is in use for pouring water through the ostomy opening the tube 24 is connected in its lower part with a conus or catheter (not shown) ensuring the correct inflow of the water, and also suitable means (likewise not shown) are provided for discharging the intestinal content, preferably into a toilet bowl.

## Claims

1. A reusable water reservoir bag (10) of a flexible plastic sheet material for use in irrigating the colon of colostomy patients, characterized in that it is provided with a thin thermometer (32) comprising encapsulated liquid crystals, the thermometer being intimately and permanently attached to the outside of the bag and being adapted to register the temperature of the water in the bag within the range of 37°C ± 10°C.

2. A water reservoir bag as claimed in claim 1, wherein the temperature of the water in the bag is registered with an accuracy of 2°C within the range stated in claim 1.

3. A water reservoir bag as claimed in claim 1 or claim 2 wherein the thermometer (32) is adapted to show the temperature registered by a colour change.

## Revendications

1. Poche à eau (12), de type réutilisable, en feuille flexible de matériau plastique, destinée à être utilisée pour l'irrigation du colon chez des patients ayant subi une colostomie, caractérisée en ce qu'elle comporte un mince thermomètre (32) constitué de cristaux liquides encapsulés, le thermomètre étant intimement et en permanence fixé sur l'extérieur de la poche et étant conçu pour enregistrer la température de l'eau dans la poche, dans la gamme de 37°C ± 10°C.

2. Poche à eau selon la revendication 1, dans laquelle la température de l'eau dans la poche est enregistrée d'une précision de 2°C dans la gamme définie dans la revendication 1.

3. Poche à eau selon la revendication 1 ou 2, dans laquelle le thermomètre (32) est conçu pour indiquer la température dans la gamme selon la revendication 1, par un changement de couleur.

## Patentansprüche

1. Wiederwendungsbare Wasserbeutel (10) aus biegsamem, dünnem Kunststoffmaterial zur Spülung des Dickdarmes von Kolostomiepatienten, dadurch gekennzeichnet, dass er ein dünnes Thermometer (32) mit eingekapselten flüssigen Kristallen aufweist, wobei das Thermometer in enger und permanenter Verbindung mit dem Äusseren des Beutels angeordnet ist und dazu eingerichtet ist, die Temperatur des Wassers im Beutel im Bereich von 37°C ± 10°C zu registrieren.

2. Wasserbeutel nach Anspruch 1, wobei die Temperatur des Wassers im Beutel mit einer Genauigkeit von 2°C innerhalb des im Anspruch 1 angegebenen Bereiches registriert wird.

3. Wasserbeutel nach Anspruch 1 oder Anspruch 2, wobei das Thermometer (32) dazu eingerichtet ist, die registrierte Temperatur durch eine Farbänderung zu zeigen.